# EUROPEAN PATENT SPECIFICATION

(11) **EP 4 534 139 B1**
(45) Date of publication and mention of the grant of the patent: **25.03.2026**
(21) Application number: 24204069.9
(22) Date of filing: 01.10.2024
(51) Int. Cl.: A61N 1/372, A61N 1/375, A61N 1/05

(54) **BIOSTIMULATOR HAVING PIVOTABLE ELECTRODE ARM**
BIOSTIMULATOR MIT SCHWENKBAREM ELEKTRODENARM
BIOSTIMULATEUR AYANT UN BRAS D'ÉLECTRODE PIVOTANT

(30) Priority: 02.10.2023 US 202363542043 P; 30.09.2024 US 202418902528
(43) Date of publication of application: 09.04.2025
(73) Proprietor: Pacesetter, Inc., Sylmar, CA 91342 (US)
(72) Inventor: Arnar, Bernhard, Sylmar, 91342 (US); Jackson, Aaron, Sylmar, 91342 (US)
(74) Representative: Barker Brettell Sweden AB

(56) References cited:
- US-A1- 2009 018 599
- US-A1- 2018 207 427
- US-A1- 2020 009 391
- US-A1- 2020 101 300

## Description

### TECHNICAL FIELD

The present disclosure relates to biostimulators and related biostimulator systems. More specifically, the present disclosure relates to leadless biostimulators and related systems useful for septal pacing.

### BACKGROUND INFORMATION

Cardiac pacing by an artificial pacemaker provides an electrical stimulation of the heart when its own natural pacemaker and/or conduction system fails to provide synchronized atrial and ventricular contractions at rates and intervals sufficient for a patient's health. Such antibradycardial pacing provides relief from symptoms and even life support for hundreds of thousands of patients. Cardiac pacing may also provide electrical overdrive stimulation to suppress or convert tachyarrhythmias, again supplying relief from symptoms and preventing or terminating arrhythmias that could lead to sudden cardiac death.

Leadless cardiac pacemakers incorporate electronic circuitry at the pacing site and eliminate leads, thereby avoiding shortcomings associated with conventional cardiac pacing systems. Leadless cardiac pacemakers can be anchored at the pacing site, e.g., in a right ventricle and, for dual-chamber pacing, in a right atrium, by an anchor. A delivery system can be used to deliver the leadless cardiac pacemakers to the target anatomy.

Cardiac pacing of the His-bundle is clinically effective and advantageous by providing a narrow QRS affecting synchronous contraction of the ventricles. His-bundle pacing in or near a membranous septum of a heart, however, has some drawbacks. The procedure is often long in duration and requires significant fluoroscopic exposure. Furthermore, successful His-bundle pacing cannot always be achieved. Pacing thresholds are often high, sensing is challenging, and success rates can be low.

Pacing at the left bundle branch (LBB) is an alternative to His-bundle pacing. Pacing at the LBB involves pacing past the His-bundle toward the right ventricular apex. More particularly, a pacing site for LBB pacing is typically below the His-bundle, on the interventricular septal wall. To achieve optimal results, the pacing site for physiological pacing at the LBB can be high on the interventricular septal wall, in the region close to the tricuspid valve and pulmonary artery outflow track. Furthermore, the pacing site may be at a depth of up to 1.5 cm within the septal wall.

US 2009/018599 A1 discloses an implantable cardiac tissue excitation system. The system includes an implantable pacing controller unit with a pulse generation circuit. The system also includes a lead with a lead body extending between a proximal lead end attachable to the pacing controller unit and a distal lead end configured to be implanted within a heart. A lead conductor extends within the lead body. The system also includes a transmitter assembly located near the distal lead end that is electrically connected to the pulse generation circuit through the lead conductor to wirelessly transmit pacing control information and pacing energy. The system also includes a leadless electrode assembly configured to be implanted within the heart that includes a receiver to receive the wireless transmission, a charge storage unit to store the charge energy, and an electrical stimulation circuit to deliver an electrical stimulus to cardiac tissue using the pacing control information and the charge energy.

US 2020/101300 A1 discloses devices and methods for a leadless implantable medical device (LIMD) comprising a housing. An electrode is disposed on the housing. An electronics circuit is disposed in the housing and is configured to sense cardiac signals, and/or generate and deliver pacing signals to the electrode. A transceiver circuit is disposed in the housing, and is configured to communicate wirelessly with an external device. A fixation antenna electrically separate from the electrode is disposed on a distal portion of the housing and is electrically coupled to the transceiver circuit, and configured to operate as an antenna for communication between the transceiver circuit and the external device.

US 2020/009391 A1 discloses a medical leadless pacemaker delivery system adapted to engage and disengage with leadless pacemakers to allow for the repeated use of the leadless pacemaker delivery system in delivering and implanting multiple leadless pacemakers into a patient heart in a serial or repeated manner. The leadless pacemaker delivery system includes a handle, an attachment mechanism, a torque portion, and a rotation limiter. The handle includes a housing. The attachment mechanism is operably coupled to the housing and configured to actuate between a released state and an engaged state. The torque portion is operably coupled to the housing and rotatable relative to the housing to transition the attachment mechanism between the released and engaged states. The torque portion includes a shaft. The rotation limiter is in sliding engagement with the shaft between a first stop and a second stop. The rotation limiter includes a first helical thread. The leadless pacemaker delivery system also includes a second helical thread in threaded engagement with the first helical thread and operably coupled to the housing. Rotation of the torque portion rotates the rotation limiter such that the first helical thread is rotated against the second helical thread, thereby driving the rotation limiter along the shaft between the first stop and the second stop.

### SUMMARY

Existing leadless pacemakers may not fit, or may interfere with heart structures, when placed at the optimal pacing site for left bundle branch (LBB) pacing. More particularly, existing leadless pacemakers have bodies that are long and rigid and, when implanted at the interventricular septal wall, could extend into contact with the cardiac tissue of a ventricular free wall or the tricuspid valve during contraction of the heart. Furthermore, a proximal end of the existing leadless pacemakers may flail within the heart chamber as the heart beats, causing cyclical contact with adjacent heart structures. Such contact could interfere with heart function. Thus, there is a need for a leadless biostimulator that can be engaged to the interventricular septal wall to pace the LBB without interfering with adjacent structures of the heart.

The invention is defined in the independent claim. Further embodiments of the invention are defined in the dependent claims. Aspects, embodiments and examples described hereinafter are only of exemplary nature and are presented for better understanding the present invention.

A biostimulator is described. In an embodiment, the biostimulator includes a body and an electrode arm. The body has an electronics compartment containing pacing circuitry. The electrode arm is pivotably coupled to the body. More particularly, the electrode arm is pivotable from an undeployed state to a deployed state. A pivot angle between the electrode arm and the body is greater in the deployed state than in the undeployed state. The electrode arm extends along an outer surface of the body in the undeployed state.

A biostimulator system is described. In an embodiment, the biostimulator system includes a biostimulator transport system. The biostimulator can be mounted on the biostimulator transport system to carry the biostimulator to or from the target anatomy. An exemplary method of pacing using the biostimulator and/or the biostimulator system is also described.

### BRIEF DESCRIPTION OF THE DRAWINGS

The features of the invention are set forth with particularity in the claims that follow. A better understanding of the features and advantages of the present disclosure will be obtained by reference to the following detailed description that sets forth illustrative embodiments, in which the principles of the disclosure are utilized, and the accompanying drawings.
FIG. 1 is a diagrammatic cross section of a patient heart illustrating an example implantation of a biostimulator in a target anatomy, in accordance with an embodiment.
FIG. 2 is a perspective view of a biostimulator system, in accordance with an embodiment.
FIG. 3 is a perspective view of a biostimulator having an electrode arm in an undeployed state, in accordance with an embodiment.
FIG. 4 is a perspective view of a biostimulator having an electrode arm in a deployed state, in accordance with an embodiment.
FIG. 5 is a perspective view of a biostimulator having a spring to bias an electrode arm away from a body, in accordance with an embodiment.
FIG. 6 is a perspective view of an end of a biostimulator having an electrical feedthrough, in accordance with an embodiment.
FIG. 7 is a flowchart of an exemplary method of implanting a biostimulator for pacing, in accordance with an embodiment.
FIG. 8 is side view of a biostimulator having an electrode arm being delivered in an undeployed state, in accordance with an embodiment.
FIG. 9 is side view of a biostimulator having an electrode arm pivoted to a deployed state, in accordance with an embodiment.

### DETAILED DESCRIPTION

Embodiments describe a biostimulator and a biostimulator system for pacing, e.g., septal pacing. The biostimulator may, however, be used in other applications, such as deep brain stimulation. Thus, reference to the biostimulator as being a cardiac pacemaker for pacing, or septal pacing, is not limiting.

In various embodiments, description is made with reference to the figures. However, certain embodiments may be practiced without one or more of these specific details, or in combination with other known methods and configurations. In the following description, numerous specific details are set forth, such as specific configurations, dimensions, and processes, in order to provide a thorough understanding of the embodiments. In other instances, well-known processes and manufacturing techniques have not been described in particular detail in order to not unnecessarily obscure the description.

The use of relative terms throughout the description may denote a relative position or direction. For example, "distal" may indicate a first direction along a longitudinal axis of a component of a biostimulator. Similarly, "proximal" may indicate a second direction opposite to the first direction. Such terms are provided to establish relative frames of reference, however, and are not intended to limit the use or orientation of a biostimulator to a specific configuration described in the various embodiments below.

In an aspect, a biostimulator includes an electrode arm pivotably coupled to a body. During deployment from an undeployed state, the electrode arm can swing outward at an angle to the body. For example, an acute pivot angle can be between the electrode arm and the body in a deployed state. The biostimulator can then be driven forward to advance the electrode arm into a target tissue, e.g., a septal wall, while the body sits flat against the septal wall. The electrode arm can therefore engage and pace the target tissue and the body can be located along the septal wall, e.g., in the ventricular apex, without interfering with a heart valve or a chamber free wall opposite to the septal wall. The biostimulator therefore fits well within the limited space of a target heart chamber, and provides long-term implant stability and reduced risk of tissue trauma.

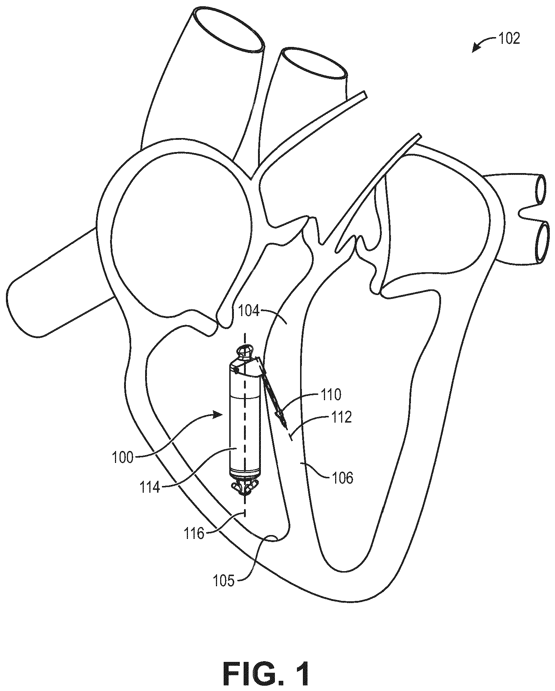

Referring to FIG. 1, a diagrammatic cross section of a patient heart illustrating an example implantation of a biostimulator in a target anatomy is shown in accordance with an embodiment. The diagram shows a biostimulator 100 attached to a patient heart 102 with a body of the biostimulator positioned toward a ventricular apex. A leadless biostimulator system, e.g., a cardiac pacing system, can include the biostimulator 100. The biostimulator 100 can be implanted in the patient heart 102, and can be leadless (and thus, may be a leadless cardiac pacemaker). The biostimulator 100 can be placed in a cardiac chamber, such as a right atrium and/or right ventricle of the heart 102, or attached to an inside or outside of the cardiac chamber. For example, the biostimulator 100 can be attached to one or more of an interventricular septal wall 104 or a ventricular apex 105 of the heart 102. More particularly, the biostimulator 100 can be delivered to an interventricular septum, and one or more elements, such as an electrode arm 110, can pierce the interventricular septal wall 104 of the septum to engage and anchor the biostimulator 100 to a target tissue 106. A body 114 of the biostimulator 100 can be directed toward the ventricular apex 105, or otherwise positioned oblique to the electrode arm 110, as described below.

The biostimulator 100 can have the electrode arm 110 having an arm axis 112, and the body 114 having a body axis 116. The arm axis 112 can be oblique to the septal wall 104 when the electrode arm 110 is affixed to the septal wall 104 and inserted into the target tissue 106. By contrast, the body axis 116 can be parallel to the wall surface of the septal wall 104 when the electrode arm 110 is affixed to the septal wall 104. For example, the arm axis 112 can be directed toward an apex wall of the ventricular apex 105 and the body 114 may be located at the ventricular apex 105.

When the electrode arm 110 is affixed to the interventricular septal wall 104, and the body 114 is located at the ventricular apex 105, the arm axis 112 and the body axis 116 can extend in different directions. For example, the arm axis 112 can extend in a direction that is transverse or oblique to a direction of the body axis 116. The non-coaxial and/or non-parallel relationship of the axes 112, 116 may be provided by a spring-loaded joint of the biostimulator 100. For example, as described below, the biostimulator 100 can include a hinge mechanism that is biased to an open position by a spring to rotate the electrode arm 110 relative to the body 114.

When the biostimulator 100 is delivered to and plunged into the septum of the heart 102, the electrode arm 110 may be positioned for deep septal pacing at a target anatomy, such as at a bundle branch in the septum. For example, as described below, an electrode of the electrode arm 110 can be positioned at a left bundle branch in the septum. The biostimulator 100 may deliver pacing impulses through the pacing electrode to the target anatomy. Accordingly, the pacing electrode can be located to effectively probe and pace the target anatomy, while the body 114 can be placed in a safe and non-obstructive location within the heart chamber.

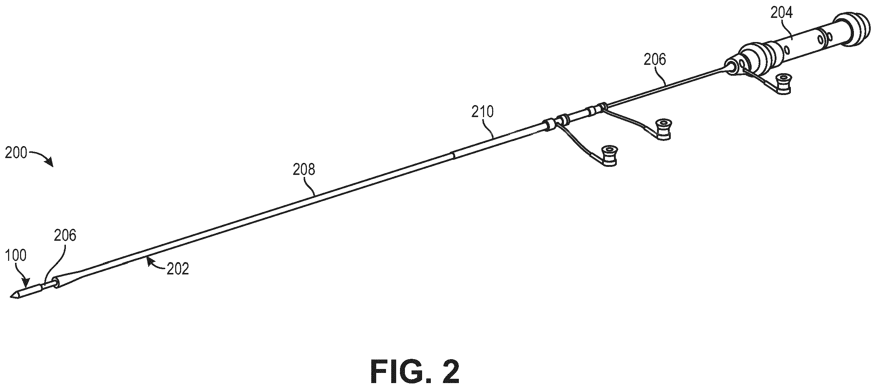

Referring to FIG. 2, a perspective view of a biostimulator system is shown in accordance with an embodiment. Leadless pacemakers or other leadless biostimulators can be delivered to or retrieved from a patient using delivery or retrieval systems. The leadless biostimulator system can include delivery or retrieval systems, which may be catheter-based systems used to carry a leadless biostimulator intravenously to or from a patient anatomy. The delivery or retrieval systems may be referred to collectively as transport systems, or biostimulator transport systems.

A biostimulator system 200 can include a biostimulator transport system 202. The biostimulator 100 can be attached, connected to, or otherwise mounted on the biostimulator transport system 202. For example, the biostimulator 100 can be mounted on a distal end of a catheter of the biostimulator transport system 202. The biostimulator 100 is thereby advanced intravenously into or out of the heart 102.

The biostimulator transport system 202 can include a handle 204 to control movement and operations of the transport system from outside of a patient anatomy. One or more elongated members extend distally from the handle 204. For example, a support member 206 can extend distally from the handle 204. The support member 206 can extend to a distal end of the transport system. In an embodiment, the biostimulator 100 is mounted on the biostimulator transport system 202, e.g., at a distal end of the support member 206.

The biostimulator transport system 202 can include a protective sleeve 208 to cover the biostimulator 100 during delivery and implantation. The protective sleeve 208 can extend over, and be longitudinally movable relative to, the support member 206. The biostimulator transport system 202 may also include an introducer sheath 210 that can extend over, and be longitudinally movable relative to, the protective sleeve 208. The introducer sheath 210 can cover a distal end of the protective sleeve 208, the support member 206, and the biostimulator 100 as those components are passed through an access device into the patient anatomy.

Several components of the biostimulator transport system 202 are described above by way of example. It will be appreciated, however, that the biostimulator transport system 202 may be configured to include additional or alternate components. More particularly, the biostimulator transport system 202 may be configured to deliver and/or retrieve the biostimulator 100 to or from the target anatomy. Delivery and/or retrieval of the biostimulator 100 can include retaining the biostimulator during transport to the target anatomy and rotation of the biostimulator during implantation of the biostimulator at the target anatomy. Accordingly, the biostimulator transport system 202 can incorporate features to retain and rotate the biostimulator 100.

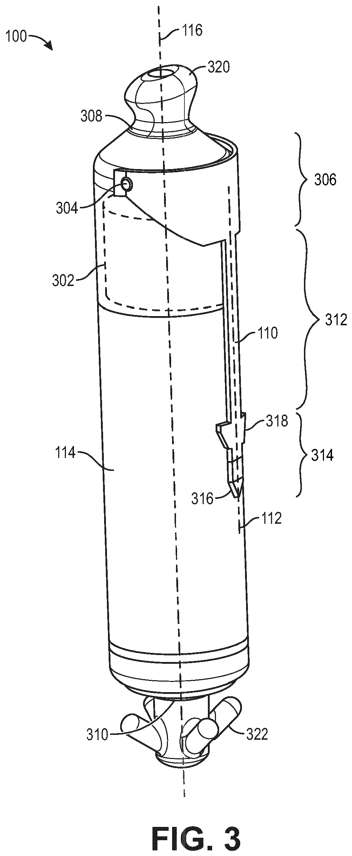

Referring to FIG. 3, a perspective view of a biostimulator having an electrode arm is shown in an undeployed state, in accordance with an embodiment. The biostimulator 100 can be a leadless cardiac pacemaker that can perform cardiac pacing and that has many of the advantages of conventional cardiac pacemakers while extending performance, functionality, and operating characteristics. In a particular embodiment, the biostimulator 100 can use two or more electrodes located on or within the body 114 of the biostimulator 100 for pacing the cardiac chamber upon receiving a triggering signal from at least one other device within the patient anatomy. The two or more electrodes of the biostimulator 100 can include an electrode of the electrode arm 110 that acts as an active electrode. The electrodes can deliver pacing pulses to target anatomies, such as bundle branches within the septum of the heart 102, to perform pacing, e.g., deep septal pacing, and optionally, can sense electrical activity from the muscle. The electrodes may also communicate bidirectionally with at least one other device within or outside the patient anatomy.

The body 114 has a longitudinal axis, e.g., the body axis 116. The body 114 can contain a primary battery to provide power for pacing, sensing, and communication, which may include, for example, bidirectional communication. The body 114 can optionally have an electronics compartment 302 (shown by hidden lines) to hold circuitry adapted for different functionality. For example, the electronics compartment 302 can contain pacing circuitry for sensing cardiac activity from the electrodes, for receiving information from at least one other device via the electrodes, for generating pacing pulses for delivery to tissue via the electrode arm 110, or other circuitry. Accordingly, the pacing circuitry can be electrically connected to the electrode arm 110. The electronics compartment 302 may contain circuits for transmitting information to at least one other device via the electrodes and can optionally contain circuits for monitoring device health. The circuitry of the biostimulator 100 can control these operations in a predetermined manner. In some implementations of a cardiac pacing system, cardiac pacing is provided without a pulse generator located in the pectoral region or abdomen, without an electrode-lead separate from the pulse generator, without a communication coil or antenna, and without an additional requirement of battery power for transmitted communication.

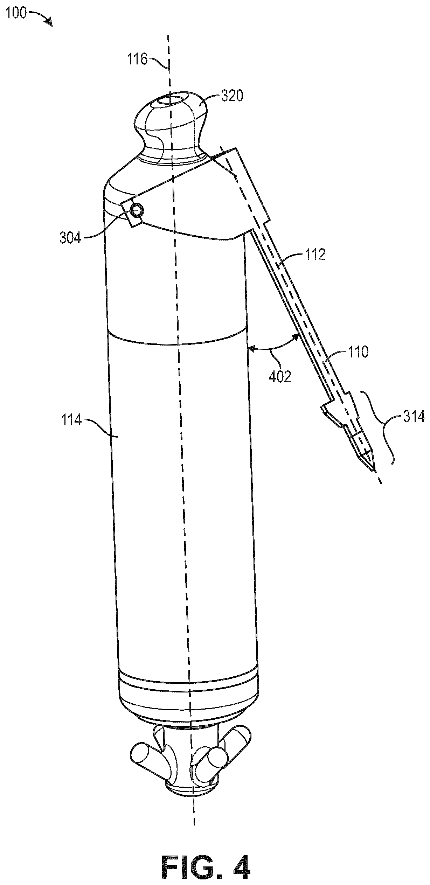

The electrode arm 110 can be pivotably coupled to the body 114. For example, a hinge 304 can connect a portion of the electrode arm 110 to the body 114. The hinge 304 can include pins extending outward from the body 114. The pins can engage corresponding holes in the electrode arm 110. The electrode arm 110 may therefore rotate about the pins to allow the electrode arm 110 to be pivotable from an undeployed state (FIG. 3) to a deployed state (FIG. 4).

The electrode arm 110 can include several portions. In an embodiment, a proximal portion 306 of the electrode arm 110 includes the holes that receive the pins of the hinge 304. The proximal portion 306 can include a partial cylinder that wraps around the body 114. More particularly, the proximal portion 306 can conform to a cylindrical outer surface of the body 114, which extends between a first body end 308 and a second body end 310. Accordingly, when the electrode arm 110 is in the undeployed state, the proximal portion 306 can fit closely against the body 114 to reduce an overall profile of the biostimulator 100.

The electrode arm 110 can include an arm segment 312 extending distally from the proximal portion 306. The arm segment 312 can include a strip of material extending longitudinally along the arm axis 112. The strip can have an aspect ratio of 10 or greater. For example, a length of the arm segment 312 can be at least 10 times a width of the arm segment.

In an embodiment, the electrode arm 110 includes an electrode tip 314. The electrode tip 314 can be at a distal end of the arm segment 312. The electrode tip 314 may be a portion of the electrode arm 110 that pierces and advances into the target tissue 106 during deployment. Accordingly, the electrode tip 314 can include a piercing tip 316 at a distal end. The piercing tip 316 can be pointed to plunge into tissue and create a path for the electrode arm 110 to extend toward the target tissue 106, e.g., the left bundle branch.

In addition to the piercing tip 316 to gain access to the target tissue 106, the electrode arm 110 may include a fixation element 318 to retain the electrode arm 110 within the target tissue 106. For example, the fixation element 318 can include one or more barbs. The barbs can include projections extending backward or laterally outward to resist extraction from the tissue. More particularly, the fixation element 318 may have a larger width than the piercing tip 316, and the width may decrease in a distal direction along the arm axis 112 such that advancement of the fixation element 318 into the target tissue 106 requires less force than retraction of the fixation element 318 from the target tissue 106.

In an embodiment, the biostimulator 100 includes an attachment feature 320 to allow the biostimulator transport system 202 to grasp and retain the biostimulator 100 in vivo. The attachment feature 320 may be mounted on the first body end 308 of the body 114. The attachment feature 320 may have a graspable shape, such as a button, a hook, a knob, or another shape that has a varying cross-sectional profile. For example, the attachment feature 320 can have a head connected to the body 114 by a neck portion. The head may have a larger cross-sectional dimension than the neck. A loop of a snare device may be placed around the neck and cinched to grab the attachment feature 320 and hold the body 114 relative to the biostimulator transport system 202.

The biostimulator 100 can include an anchor element 322 to secure the body 114 to a heart structure. In an embodiment, the anchor element 322 is mounted on the second body end 310 of the body 114. More particularly, the anchor element 322 can be mounted on the body 114 opposite from the attachment feature 320. For example, the attachment feature 320 may be at a proximal end of the body 114 to be near and exposed to the biostimulator transport system 202. By contrast, the anchor element 322 may be at a distal end of the body 114 to be closer to a ventricular apex 105 when the electrode arm 110 is plunged into the septal wall 104. The anchor element 322 can include one or more prongs extending radially outward from the body 114. The prongs may also extend in the proximal direction. The prongs may therefore entangle with heart structures in the ventricular apex 105, e.g., chordae tendinae, to secure the distal end of the body 114 within the ventricular apex 105 away from the ventricular free wall or other sensitive structures of the heart chamber.

The electrode arm 110 can be separated from the body 114 by a pivot angle. In the undeployed state, the electrode arm 110 can rest against the outer surface of the body 114 and, thus, the pivot angle may effectively be 0 degrees. More particularly, the arm axis 112 of the electrode arm 110 can be parallel to the body axis 116 of the body 114 in the undeployed state. As described above, electrode arm 110 is pivotable relative to the body 114 and, thus, the pivot angle may increase as electrode arm 110 pivots outward to be separated farther from the outer surface of the body 114.

Referring to FIG. 4, a perspective view of a biostimulator having an electrode arm in a deployed state is shown in accordance with an embodiment. As described above, a pivot angle 402 between the electrode arm 110 and the body 114 can increase or decrease as the electrode arm pivots relative to the body 114. When the electrode arm 110 pivots outward at the hinge 304, the pivot angle 402 can increase. Accordingly, when the electrode arm 110 pivots to a deployed state, as shown in FIG. 4, the pivot angle 402 may be greater than in the undeployed state shown in FIG. 3.

In the deployed state, the arm axis 112, along which the electrode arm 110 including arm segment 312 (see FIG. 3) extends, is redirected to extend in a direction that is not parallel to the body axis 116. The pivot angle 402 may be defined between the arm axis 112 and the body axis 116. Outward movement of the electrode arm 110 from the body 114 may be constrained to maintain the pivot angle 402 at an acute angle. More particularly, the pivot angle 402 may be an acute angle both in the undeployed state and the deployed state. The pivot angle 402 can be limited, for example, by a stop incorporated in the hinge 304, contact between the proximal portion 306 (see FIG. 3) of the electrode arm 110 and the body 114, or another motion-limiting feature of the biostimulator 100. In any case, movement of the electrode arm 110 relative to the body 114 may be constrained within an angle that is no more than 55 degrees. For example, the maximum pivot angle 402 may be within a range of 35-55 degrees. In an embodiment, the pivot angle 402 is constrained to a predetermined angle, such as 45 degrees. The predetermined angle can be set to allow the electrode arm 110 to engage and be pressed downward into the target tissue 106 at an angle that facilitates placement of the electrode tip 314 at a desired pacing site. The electrode arm 110 may therefore be pushed into the tissue to a depth where the pacing site, e.g., the left bundle branch, can be effectively paced.

In the deployed state, the arm axis 112 is oblique to the body axis 116. As a result, the electrode tip 314 can be positioned radially outward of the outer surface of the body 114. The oblique angle allows the electrode tip 314 to plunge into the target tissue 106 at an angle that is oblique to a septal wall surface defining the heart chamber. Accordingly, the electrode arm 110 can be directed downward and outward within the septal wall 104 while the body 114 slides downward along the septal wall surface.

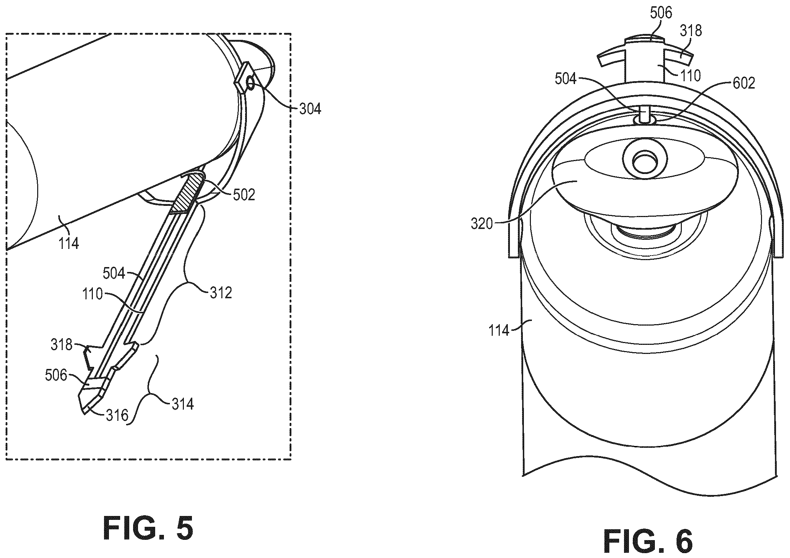

Referring to FIG. 5, a perspective view of a biostimulator having a spring to bias an electrode arm away from a body is shown in accordance with an embodiment. The electrode arm 110 can be spring-loaded. More particularly, the biostimulator 100 can include a spring 502 to bias the electrode arm 110 toward the deployed state. The spring 502 may be collapsed to move the electrode arm 110 toward the body 114 to the undeployed state. Accordingly, the electrode arm 110 can be folded inward to load the biostimulator 100 into the biostimulator transport system 202, and upon deployment from the biostimulator transport system 202, the spring-activated electrode arm 110 can swing outward to the deployed state. As described above, electrode arm 110 can actuate to a predetermined angle, e.g., an acute angle no more than 55 degrees. The predetermined angle can be limited by a stop, or by an extension range of the spring 502.

Springs 502 of different types may be used to achieve the spring-loaded function of the biostimulator 100. In an embodiment, the spring 502 includes a flexible tab positioned between the body 114 and the electrode arm 110. For example, the tab can have an end attached or disposed against the body 114 and an end attached or disposed against electrode arm 110. The ends can be squeezed together to cause a flexible tab to bend and collapse into the undeployed state. When the squeezing force is removed, the flexible tab can resiliently expand, allowing the ends to separate and pivot the electrode arm 110 outward about the hinge 304. In an alternate embodiment, the biostimulator 100 may incorporate a torsion spring 502 having an end connected to the body 114 and an end connected to the electrode arm 110. The torsion spring 502 can wind up to collapse into the undeployed state or unwind to move the electrode arm 110 outward relative to the body 114.

The electrode arm 110 can include an electrical pathway to conduct pacing impulses from pacing circuitry within the body 114 to the electrode tip 314. In an embodiment, the biostimulator 100 includes an electrical lead 504 extending along the electrode arm 110 to the electrode tip 314. For example, the electrical lead 504 can extend to an electrode 506 positioned on the electrode arm 110 near the piercing tip 316. The electrode 506 may be longitudinally between the piercing tip 316 and the fixation element 318 of electrode tip 314. Accordingly, the electrical lead 504 can transmit the pacing impulses to tissue that the electrode tip 314 is anchored within.

In an embodiment, the electrical lead 504 can extend along an inner surface of the electrode arm 110. More particularly, the electrical lead 504 may run along the inner surface of the electrode arm 110 facing the outer surface of the body 114. Electrical lead 504 may therefore be between the arm segment 312 and the body 114. Electrical lead 504 may be insulated from the arm segment 312, e.g., by a thin layer of dielectric film. Accordingly, the electrical path along the electrode arm 110 may be well-defined to efficiently transfer pacing impulses to the electrode 506.

Referring to FIG. 6, a perspective view of an end of a biostimulator having an electrical feedthrough is shown in accordance with an embodiment. The electrical pathway carrying pacing impulses to the electrode tip 314 may include a passage through the body 114. More particularly, the biostimulator 100 can include an electrical feedthrough 602 in the body 114. The electrical lead 504 can be electrically connected to the electrical feedthrough 602 to receive the pacing impulses from the pacing circuitry within the electronics compartment 302. The pacing impulses can therefore travel through the body 114 and along the electrode arm 110 to the electrode 506, which may be located distal to the fixation element 318. More particularly, the pacing impulses can be delivered to the target tissue 106 in which the electrode arm 110 is anchored.

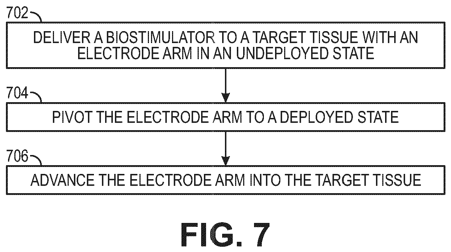

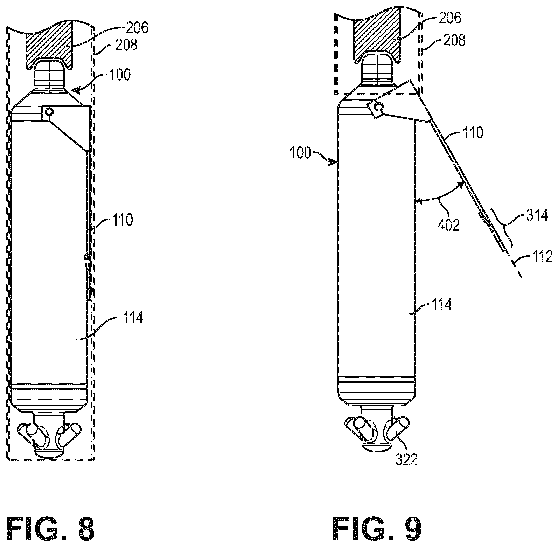

Referring to FIG. 7, a flowchart of an exemplary method of implanting a biostimulator for pacing is shown in accordance with an embodiment. Operations of the method are shown in FIGS. 8-9. Accordingly, FIGS. 7-9 are alternately referred to in the description below.

Referring to FIG. 8, a side view of a biostimulator having an electrode arm being delivered in an undeployed state is shown in accordance with an embodiment. At operation 702, the biostimulator 100 is delivered to the target tissue 106 with the electrode arm 110 in the undeployed state. The electrode arm 110 can be collapsed against the outer surface of the body 114. In an embodiment, when the electrode arm 110 is squeezed inward against the body 114, the biostimulator 100 is loaded into the biostimulator transport system 202. For example, the biostimulator 100 can be housed within the protective sleeve 208 of the biostimulator transport system 202. The protective sleeve 208 can have an inner dimension that fits the biostimulator 100 when the electrode arm 110 is collapsed. The biostimulator transport system 202 can therefore be tracked through the vasculature and advanced toward the target tissue 106.

Referring to FIG. 9, a side view of a biostimulator having an electrode arm pivoted to a deployed state is shown in accordance with an embodiment. At operation 704, the electrode arm 110 is pivoted to the deployed state. When the distal end of the biostimulator transport system 202 is located adjacent to the target tissue 106, such as the septal wall 104, the protective sleeve 208 can be retracted to expose the electrode arm 110. When the electrode arm 110 becomes exposed, the spring-loaded hinge can actuate to cause the electrode arm 110 to swing outward to the deployed state. In the deployed state, the pivot angle 402 between the electrode arm 110 and the body 114 is greater than in the undeployed state. Accordingly, pivoting the electrode arm 110 redirects the arm axis 112 of the electrode arm 110 toward the target tissue 106, e.g., the left bundle branch.

At operation 706, the electrode arm 110 is advanced into the target tissue 106. For example, the support member 206 of the biostimulator transport system 202 can engage the biostimulator 100 to push the electrode arm 110 forward. Electrode arm 110 can plunge into the septal wall 104 to locate the electrode 506 at the target tissue 106. The body 114, on the other hand, may slide downward along the septal wall 104 to be located within the ventricular apex 105 (FIG. 1), for example. The anchor element 322 may secure the biostimulator end within the apical region to hold the body 114 against the septal wall 104, out of the way of sensitive heart structures.

When the biostimulator 100 is implanted with the electrode tip 314 at the target tissue 106, pacing impulses can be delivered to the electrode 506. The pacing impulses can travel from the pacing circuitry, through the electrical pathway including the electrical lead 504, to the electrode 506. The biostimulator 100 may therefore effectively pace the target tissue 106 within which the electrode 506 is implanted.

## Claims

1. A biostimulator (100), comprising:
a body (114) having an electronics compartment (302) containing pacing circuitry; and
an electrode arm (110) pivotably coupled to the body (114), wherein the electrode arm (110) is pivotable from an undeployed state to a deployed state, and wherein a pivot angle (402) between the electrode (110) arm and the body (114) is greater in the deployed state than in the undeployed state, **characterized in that** the electrode arm (110) extends along an outer surface of the body (114) in the undeployed state.

2. The biostimulator of claim 1, wherein the pivot angle (402) is an acute angle in the deployed state.

3. The biostimulator of claim 2, wherein the acute angle is no more than 55 degrees.

4. The biostimulator of any one of claims 1 to 3, wherein an arm axis (112) of the electrode arm (110) is parallel to a body axis (116) of the body (114) in the undeployed state, and wherein the arm axis (112) is oblique to the body axis (116) in the deployed state.

5. The biostimulator of any one of claims 1 to 4, wherein the electrode arm (110) includes an electrode tip (314) having a fixation element (318).

6. The biostimulator of claim 5, wherein the fixation element (318) includes one or more barbs.

7. The biostimulator of claim 5 or 6, further comprising an electrical lead (504) extending along the electrode arm (110) to the electrode tip (314).

8. The biostimulator of claim 7, further comprising an electrical feedthrough (602) in the body (114), wherein the electrical lead (504) is electrically connected to the electrical feedthrough (602) to receive pacing impulses from the pacing circuitry.

9. The biostimulator of any one of claims 1 to 8, further comprising a spring (502) to bias the electrode arm (110) toward the deployed state.

10. The biostimulator of claim 9, wherein the spring (502) is a flexible tab positioned between the body (114) and the electrode arm (110).

11. The biostimulator of claim 9, wherein the spring (502) includes a torsion spring having a first end connected to the body (114) and a second end connected to the electrode arm (110).

12. The biostimulator of any one of claims 1 to 11, further comprising an attachment feature (320) mounted on a first body end (308) of the body (114).

13. The biostimulator of claim 12, further comprising an anchor element (322) mounted on a second body end (310) of the body (114) opposite from the attachment feature (320).

14. A biostimulator system (200), comprising:
a biostimulator transport system (202); and
a biostimulator (100) of any one of claims 1 to 13, wherein the biostimulator (100) is mounted on the biostimulator transport system (202).

15. The biostimulator system of claim 14, wherein the biostimulator transport system (202) includes a support member (206) to engage the biostimulator (100) and to push the electrode arm (110) forward.

## Patentansprüche

1. Biostimulator (100), umfassend:
einen Körper (114), der ein Elektronikfach (302) aufweist, das eine Schrittmacherschaltung enthält; und
einen Elektrodenarm (110), der schwenkbar an den Körper (114) gekoppelt ist, wobei der Elektrodenarm (110) von einem nicht eingesetzten Zustand in einen eingesetzten Zustand schwenkbar ist und wobei ein Schwenkwinkel (402) zwischen dem Elektroden (110)arm und dem Körper (114) in dem eingesetzten Zustand größer als in dem nicht eingesetzten Zustand ist, **dadurch gekennzeichnet, dass** sich der Elektrodenarm (110) in dem nicht eingesetzten Zustand entlang einer äußeren Oberfläche des Körpers (114) erstreckt.

2. Biostimulator nach Anspruch 1, wobei der Schwenkwinkel (402) in dem eingesetzten Zustand ein spitzer Winkel ist.

3. Biostimulator nach Anspruch 2, wobei der spitze Winkel nicht mehr als 55 Grad beträgt.

4. Biostimulator nach einem der Ansprüche 1 bis 3, wobei in dem nicht eingesetzten Zustand eine Armachse (112) des Elektrodenarms (110) parallel zu einer Körperachse (116) des Körpers (114) ist und wobei in dem eingesetzten Zustand die Armachse (112) schräg zu der Körperachse (116) ist.

5. Biostimulator nach einem der Ansprüche 1 bis 4, wobei der Elektrodenarm (110) eine Elektrodenspitze (314) beinhaltet, die ein Befestigungselement (318) aufweist.

6. Biostimulator nach Anspruch 5, wobei das Befestigungselement (318) einen oder mehrere Stachel beinhaltet.

7. Biostimulator nach Anspruch 5 oder 6, ferner umfassend einen elektrischen Anschluss (504), der sich entlang des Elektrodenarms (110) zu der Elektrodenspitze (314) erstreckt.

8. Biostimulator nach Anspruch 7, ferner umfassend eine elektrische Durchführung (602) in dem Körper (114), wobei der elektrische Anschluss (504) elektrisch mit der elektrischen Durchführung (602) verbunden ist, um Schrittmacherimpulse von der Schrittmacherschaltung zu empfangen.

9. Biostimulator nach einem der Ansprüche 1 bis 8, ferner umfassend eine Feder (502), um den Elektrodenarm (110) in den eingesetzten Zustand vorzuspannen.

10. Biostimulator nach Anspruch 9, wobei die Feder (502) eine flexible Lasche ist, die zwischen dem Körper (114) und dem Elektrodenarm (110) positioniert ist.

11. Biostimulator nach Anspruch 9, wobei die Feder (502) eine Torsionsfeder beinhaltet, die ein erstes Ende, das mit dem Körper (114) verbunden ist, und ein zweites Ende, das mit dem Elektrodenarm (110) verbunden ist, beinhaltet.

12. Biostimulator nach einem der Ansprüche 1 bis 11, ferner umfassend ein Befestigungsmerkmal (320), das an einem ersten Körperende (308) des Körpers (114) montiert ist.

13. Biostimulator nach Anspruch 12, ferner umfassend ein Ankerelement (322), das an einem zweiten Körperende (310) des Körpers (114) gegenüber dem Befestigungsmerkmal (320) montiert ist.

14. Biostimulatorsystem (200), umfassend:
ein Biostimulatortransportsystem (202); und
einen Biostimulator (100) nach einem der Ansprüche 1 bis 13, wobei der Biostimulator (100) an dem Biostimulatortransportsystem (202) montiert ist.

15. Biostimulatorsystem nach Anspruch 14, wobei das Biostimulatortransportsystem (202) eine Stützkomponente (206) beinhaltet, die in den Biostimulator (100) eingreift und den Elektrodenarm (110) nach vorne drückt.

## Revendications

1. Biostimulateur (100), comprenant :
un corps (114) ayant un compartiment électronique (302) contenant un circuit de stimulation ; et
un bras d'électrode (110) couplé de manière pivotante au corps (114), dans lequel le bras d'électrode (110) peut pivoter à partir d'un état non déployé vers un état déployé, et dans lequel un angle de pivotement (402) entre le bras d'électrode (110) et le corps (114) est plus grand dans l'état déployé que dans l'état non déployé, **caractérisé en ce que** le bras d'électrode (110) s'étend le long d'une surface externe du corps (114) dans l'état non déployé.

2. Biostimulateur selon la revendication 1, dans lequel l'angle de pivotement (402) est un angle aigu dans l'état déployé.

3. Biostimulateur selon la revendication 2, dans lequel l'angle aigu n'est pas supérieur à 55 degrés.

4. Biostimulateur selon l'une quelconque des revendications 1 à 3, dans lequel un axe de bras (112) du bras d'électrode (110) est parallèle à un axe de corps (116) du corps (114) dans l'état non déployé, et dans lequel l'axe de bras (112) est oblique par rapport à l'axe de corps (116) dans l'état déployé.

5. Biostimulateur selon l'une quelconque des revendications 1 à 4, dans lequel le bras d'électrode (110) inclut une pointe d'électrode (314) ayant un élément de fixation (318).

6. Biostimulateur selon la revendication 5, dans lequel l'élément de fixation (318) inclut une ou plusieurs barbes.

7. Biostimulateur selon la revendication 5 ou 6, comprenant en outre un fil électrique (504) s'étendant le long du bras d'électrode (110) jusqu'à la pointe d'électrode (314).

8. Biostimulateur selon la revendication 7, comprenant en outre une traversée électrique (602) dans le corps (114), dans lequel le fil électrique (504) est connecté électriquement à la traversée électrique (602) pour recevoir des impulsions de stimulation à partir du circuit de stimulation.

9. Biostimulateur selon l'une quelconque des revendications 1 à 8, comprenant en outre un ressort (502) pour solliciter le bras d'électrode (110) vers l'état déployé.

10. Biostimulateur selon la revendication 9, dans lequel le ressort (502) est une languette flexible positionnée entre le corps (114) et le bras d'électrode (110).

11. Biostimulateur selon la revendication 9, dans lequel le ressort (502) inclut un ressort de torsion ayant une première extrémité connectée au corps (114) et une seconde extrémité connectée au bras d'électrode (110).

12. Biostimulateur selon l'une quelconque des revendications 1 à 11, comprenant en outre une structure de fixation (320) montée sur une première extrémité de corps (308) du corps (114).

13. Biostimulateur selon la revendication 12, comprenant en outre un élément d'ancrage (322) monté sur une seconde extrémité de corps (310) du corps (114) opposée à la structure de fixation (320).

14. Système de biostimulateur (200), comprenant :
un système de transport de biostimulateur (202) ; et
un biostimulateur (100) selon l'une quelconque des revendications 1 à 13, dans lequel le biostimulateur (100) est monté sur le système de transport de biostimulateur (202).

15. Système de biostimulateur selon la revendication 14, dans lequel le système de transport de biostimulateur (202) inclut un élément de support (206) pour venir en prise avec le biostimulateur (100) et pour pousser le bras d'électrode (110) vers l'avant.
